# EUROPEAN PATENT APPLICATION

(11) **EP 4 174 048 A1**
(43) Date of publication of application: **03.05.2023**
(21) Application number: 21205656.8
(22) Date of filing: 29.10.2021
(51) Int. Cl.: C07C 7/00, C07C 7/10, C07C 7/171, C07C 9/16

(54) **PROCES FOR TREATING A COMPOSITION CONTAINING RENEWABLE ISODODECANE**

(71) Applicant: Haltermann Carless Deutschland GmbH, 21107 Hamburg (DE)
(72) Inventor: RULHOFF, Sacha, 21107 Hamburg (DE); GRÖNWOLDT, Christina, 21107 Hamburg (DE); BECK, Janina, 21107 Hamburg (DE)
(74) Representative: Hoefer & Partner Patentanwälte mbB

(57) **Abstract**

The present invention concerns a process for treating a composition containing renewable isododecane, the process comprising inter alia treating a first composition with Oleum; adding water to the treated first composition and agitating the mixture; allowing the aqueous phase containing oxidized and/or sulfonized compounds and the non-aqueous phase containing renewable isododecane to separate; removing the aqueous phase containing oxidized and/or sulfonated compounds in order to obtain the non-aqueous phase containing the renewable isododecane; adding an alkaline aqueous washing solution to the non-aqueous phase containing the renewable isododecane; removing the aqueous washing solution and optionally drying the non-aqueous phase containing the renewable isododecane in order to obtain a second composition containing renewable isododecane. The invention further concerns a composition obtainable by this process and its use, in particular in a cosmetic product of application.

## Description

### Field of the Invention

The present invention concerns a process for treating a composition containing renewable isododecane, in order to obtain a high-quality product which can be used in particular in the cosmetic and pharma industry. The process comprises inter alia a specific treatment with Oleum. The composition obtainable by using this process is of high quality and can be used e.g. in a cosmetic product or application.

### Background of the invention

Isododecane is a branched C12 hydrocarbon for which 355 different isomers exist. The most important and well-known isomer is 2,2,4,6,6-Pentamethylheptane. Isododecane acts as a reaction medium used for polymerization reactions. It is also useful as an emollient and solvent in skin care products due to its high spreadability, low viscosity and density. It is often used in anti-aging serums and also useful in many different cosmetic products such as eyeliner, hair care, hair sprays, perfume, conditioners and lotions.

For use in the cosmetics and pharma industry, compositions comprising isododecane have to fulfil high quality requirements. They are commonly derived from fossil oil.

WO2011/140560 A1 is directed to methods for preparing a renewable jet fuel blendstock, and blendstocks prepared by such methods, comprising fermenting a biomass-derived feedstock to form one or more C2-C6 alcohols such as isobutanol, catalytically dehydrate and oligomerize the alcohols to form higher molecular weight olefins (e.g., C8-C16 olefins), and hydrogenating at least a portion of the higher molecular weight olefins to form a renewable jet fuel blendstock comprising C12 and C16 isoparaffins. The content of the C12 isoparaffins (isododecane) may be adjusted by conventional distillation.

There exists a need for a treatment process allowing the efficient and simple preparation of high-quality compositions containing isododecane also from renewable sources, meeting the requirements of the cosmetic and pharmaceutical industry and avoiding or reducing the content of unwanted impurities. The present invention addresses these and further objects.

### Summary and Details of the Invention

A first aspect of the present invention is directed to a process for treating a composition containing renewable isododecane, the process comprising the following steps:
a) Selecting a first composition containing renewable isododecane, in particular at least 20% renewable isododecane;
b) Optionally increasing the content of renewable isododecane in the first composition to at least 80%, in particular at least 85%;
c) Treating (or contacting) the first composition with Oleum;
d) Adding water to the treated first composition of step c) and agitating the mixture;
e) Allowing the aqueous phase containing oxidized and/or sulfonized compounds and the non-aqueous phase containing renewable isododecane to separate;
f) Removing the aqueous phase containing oxidized and/or sulfonated compounds in order to obtain the non-aqueous phase containing the renewable isododecane;
g) Adding an alkaline aqueous washing solution to the non-aqueous phase containing the renewable isododecane;
h) removing the aqueous washing solution of step g), optionally repeating the washing step with alkaline aqueous washing solution and/or water, and optionally drying the non-aqueous phase containing the renewable isododecane, in order to obtain a second composition containing renewable isododecane;
i) Optionally repeating steps c) to h) using the non-aqueous phase containing the renewable isododecane obtained in step h).

It has been surprisingly found, that the treatment with Oleum within a process according to the invention is highly efficient in producing high-quality isododecane compositions with reduced levels of unwanted impurities, and at the same time allows to also improve the optical appearance (absence of any unwanted coloration) of the composition. Unexpectedly, the treatment process as described also helps to avoid any unpleasant or strongly paraffinic odour and allows providing compositions with a light, pleasant odour which are highly acceptable to the cosmetic and pharmaceutical industry. In a preferred embodiment of the process of the invention, the step of treating the first composition with Oleum is repeated, i.e. the Oleum first added is removed, and fresh Oleum is added.

According to the present invention, preferably a composition containing at least 20% isododecane, in particular renewable isododecane, is used as a starting material, designated "first composition" herein. Such compositions are known in the art, both from the processing of fossil and renewable sources. For example, WO2011/140560 A1 describes the preparation of compositions containing renewable isododecane (C12 isoparaffins). Thus, in one embodiment, a first composition can be obtained as described in WO2011/140560 A1 by methods for preparing a renewable jet fuel blendstock, and blendstocks prepared by such methods, comprising fermenting a biomass-derived feedstock to form one or more C2-C6 alcohols such as ethanol or isobutanol, catalytically dehydrate and oligomerize the alcohols to form higher molecular weight olefins (e.g., C8-C16 olefins), and hydrogenating at least a portion of the higher molecular weight olefins to form a renewable jet fuel blendstock comprising C12 and C16 isoparaffins.

In many cases, the content of isododecane in the first composition will preferably be at least 40% or higher, more preferably 80% or higher, in particular 85% or higher. If it is desired (according to optional step b) above) to increase the content of (renewable) isododecane in the first composition to at least 80%, in particular at least 85%, conventional distillation or other fractionation steps known to the skilled person can be used. In a preferred embodiment of the invention, step b) comprises or is a fractionation with a boiling range of about 170°C - 190°C, in particular about 172°C - 185°C, more particular about 174°C - 183°C. In certain cases, the content of isododecane in the first composition might however be even lower than 20%, even 10% or lower. Thus, according to one particular aspect of the invention, a composition containing less than 20% isododecane, in particular 10% or less isododecane, is used as a starting material or first composition.

According to a preferred embodiment of the present invention, the time period between the addition of water according to step d) and the removal of the aqueous phase according to step f) is at least 5 hours, preferably at least 10 hours, in particular at least 15 hours, more particular at least 20 hours. Thus, it has been found that such a long incubation period surprisingly improves the odour, purity and appearance of the resulting (second) composition.

It was also unexpectedly found, that the results of the process of the invention are particularly advantageous and an optimum separation, also of sulfonic acids is obtained if the amount of water added in step d) is less than 5 vol./vol. %, preferably less than 3 vol./vol. % of the treated first composition of step c), i.e. after removal of the (spent) Oleum. Further preferred, the amount of water added in step d) is at least 0.001 vol./vol. %, preferably 0.001 vol./vol. % of the treated first composition of step c), i.e. after removal of the (spent) Oleum. In a particularly preferred embodiment of the invention, the amount of water added in step d) is from 0.001 vol./vol. % (in particular 0.01 vol./vol. %) to 5 vol./vol. %.

In step g) an alkaline aqueous washing solution is added to the non-aqueous phase containing the renewable isododecane. The alkaline aqueous washing solution is preferably a 0.05 to 0.4 molar aqueous solution of a strong base, such as NaOH. Preferably, about 0.5% to 5% of the alkaline aqueous washing solution are added, based on the weight of the non-aqueous phase containing the renewable isododecane.

In step h), the (alkaline) aqueous washing solution is removed, in order to obtain a second composition containing renewable isododecane. Step g) (i.e. the addition of an alkaline washing solution) and step h) (i.e. the removal of the (alkaline) aqueous washing solution) may preferably be repeated. Further preferred, one or more washing steps with water may be performed. The absence of unwanted amounts of residual water in the final second composition can be ensured by standard means known to the skilled person, i.e. optionally a drying step is included.

According to a further, preferred embodiment of the present invention, 0.5 to 5 % of Oleum, are used in step c) above, based on the total mass of the first composition.

In general, Step b) comprises also the separation of the (treated) first composition from the Oleum (i.e. in a separation funnel or the like) before the addition of water in step d). According to a further, preferred embodiment of the present invention, step c) of treating the first composition with Oleum comprises more than one addition of Oleum to the first composition, i.e. the addition of (treatment with) Oleum (and its subsequent separation) is repeated, in particular twice.

"Oleum" as used herein is known to the skilled person and is also called "fuming sulphuric acid". It is a term referring to solutions of various compositions of sulfur trioxide in sulfuric acid, or sometimes more specifically to disulfuric acid (also known as pyrosulfuric acid). According to one embodiment, Oleum is identified by the CAS number 8014-95-7. Oleum can be described by the formula ySO₃·H2O where y is the total molar mass of sulfur trioxide content. The value of y can be varied, to include different Oleums. They can also be described by the formula H2SO₄·xSO₃ where x is now defined as the molar free sulfur trioxide content. Oleum is generally assessed according to the free SO₃ content by mass.

According to a preferred embodiment of the invention, the Oleum has at least 5%, preferably at least 10%, in particular about 20% free mass of SO₃.

As stated above, compositions containing isododecane, in particular renewable isododecane which may be used as first compositions according to the present invention are known in the art and can be easily prepared by fractionation of hydrocarbon feeds of renewable sources. However, those known compositions were not usable or optimized for use in cosmetic or pharmaceutical products due to their impurity levels, their colour and/or their odour.

According to a preferred aspect of the present invention, the first composition comprises:
at least 95% isoparaffins,
not more than 5% n-paraffins,
at least 40%, preferably at least 50% isododecane,
not more than 10% C8 isoparaffins,
not more than 40% C16 isoparaffins,
not more than 5%, preferably not more than 3%, in particular not more than 1% aromatics not more than 1% naphthenes,
no more than 5%, preferably not more than 3%, in particular not more than 1% hydrocarbons comprising heteroatoms, in particular comprising oxygen, nitrogen or sulphur,
not more than 2 wt.-%, preferably less than 1 wt.-%, in particular less than 2000 mg/kg (2000 ppmw) alkenes and alkynes, as defined by bromine index.

It is generally preferred that the isododecane present in the first composition comprises at least 60%, preferably at least 70%, in particular at least 75% 2,2,4,6,6-Pentamethylheptane.

Following the treatment process according to the invention as described herein, the second composition comprises preferably:
less than 200 ppm, in particular less than 50 ppm aromatics,
less than 200ppm hydrocarbons comprising heteroatoms, in particular comprising oxygen, nitrogen or sulphur,
less than 1 ppm sulphur,
no more than 1% hydrocarbons comprising heteroatoms, in particular comprising oxygen, nitrogen or sulfur;
less than 600 mg/kg alkenes and alkynes, as defined by bromine index.

According to a further preferred aspect of the present invention, the second composition (further) comprises:
at least 98% isoparaffins,
not more than 2% n-paraffins,
less than 1% C8 isoparaffins,
at least 80% isododecane, preferably at least 98% thereof 2,2,4,6,6-Pentamethylheptane,
not more than 2% C16 isoparaffins,
not more than 2% paraffins C11 or smaller,
not more than 1% naphthenes, in particular less than 50 ppm.

According to a further preferred embodiment, the first composition is a renewable jet fuel blendstock, in particular having the composition as set out above for the first composition. Such a jet fuel blendstock can for example be prepared according to the disclosure of WO2011/140560A1, incorporated herein by reference.

Thus, the preparation may comprise: (a) treating biomass to form a feedstock; (b) fermenting the feedstock with one or more species of microorganism, thereby forming isobutanol; (c) dehydrating at least a portion of the isobutanol obtained in step (b), thereby forming a dehydration product comprising isobutene; (d) oligomerizing at least a portion of the dehydration product, thereby forming a product comprising one or more Cs, one or more C12, and one or more C16 unsaturated oligomers; and (e) hydrogenating at least a portion of the product of step (d) in the presence of hydrogen, thereby forming a renewable jet fuel blendstock comprising one or more C12 and one or more C16 saturated alkanes; (f) adjusting the ratio of C12 and C16 saturated alkanes provided by step (e).

In detail, first renewable alcohols (e.g., ethanol or isobutanol) are formed from biomass, see for example paragraphs [0060] to [0068] of WO2011/140560A1 explicitly incorporated herein by reference. This can be done by any combination of methods including fermentation, thermochemical (e.g., Fischer-Tropsch), photosynthesis, etc. The feedstock for a fermentation process can be any suitable fermentable feedstock known in the art, for example sugars derived from agricultural crops such as sugarcane, corn, etc. Alternatively, the feedstock for fermentation can be prepared by the hydrolysis of biomass, for example lignocellulosic biomass (e.g. wood, corn stover, switchgrass, herbiage plants, ocean biomass, etc.). The lignocellulosic biomass can be converted to fermentable sugars by various processes known in the art.

The renewable alcohols (e.g., ethanol or isobutanol) are then isolated by methods known in the art, and dehydrated to olefins (e.g. ethylene or butenes), see for example paragraphs [0069] to [0070] and [0071] to [0074] of WO2011/140560A1,respectively, explicitly incorporated herein by reference.

Butenes/olefins so produced may be reacted over a heterogeneous acidic catalyst, such as sulfonic acid resin, solid phosphoric acid, or acidic zeolite, or any other suitable catalyst at moderate temperatures (e.g., 100-300°C) and pressures (e.g., 0-1000 psig) to form a blend of C8-C16 oligomers of butene(s), and the content of C12/C16 olefinic oligomers is maximized, see for example paragraphs [0075] to [0086] of WO2011/140560A1 also explicitly incorporated herein by reference.

Subsequently, the C12 and C16 olefins are subjected to a hydrogenation/hydrotreating, and separation by conventional methods (see for example paragraphs [0087] to [0100] of WO2011/140560A1 explicitly incorporated herein by reference), followed by a separation of the resulting C12 and C16 isoparaffins (see for example paragraph [0101] of WO2011/140560A1 explicitly incorporated herein by reference). Such a composition having the desired renewable isododecane content may be used as a first composition in the process according to the present invention.

The present inventors have found that a hydrogenation as e.g. described in WO2011/140560A1, see above, is disadvantageous, and the process of the present invention using a defined oleum treatment and procedure provides unexpected advantages. In hydrogenation, aromatics like benzene or toluene are converted to cyclohexane resp. methylcyclohexane. These now naphthenes called substances stay in the hydrocarbon matrix and are notoriously difficult to remove. By gas chromatography, using the right method and standards, these are easily detectable. Distillation cannot remove the naphthenes entirely as they are too similar in boiling point and chemical behavior to n-paraffins and iso-paraffins.

The treatment with oleum (sulfur trioxide, dissolved in concentrated sulfuric acid, see above) according to the present invention was found to allow straightforward preparation of a high quality, colorless composition with a pleasant odour. The concentrated acid oxidizes olefins, hetero-compounds and other unsaturated molecules. Aromatics are not directly oxidized but sulfonated, yielding sulfonic acids. All these compounds are water-soluble, especially in slightly alkaline aqueous solutions. This means, all unwanted compounds are completely removed from the paraffin matrix and do not remain as saturated molecules like naphthenes. This is detectable in gas chromatography. Thus, the process of the invention as described herein provides a more suitable product for sensitive applications like cosmetics and pharma.

A further aspect of the present invention is directed to a composition containing renewable isododecane, obtainable by the process of the invention as described herein. It was surprisingly found that such compositions obtained by the inventive process as described herein have a very high quality with low contents of unwanted impurities, and provide an optimal colourless appearance as well as a light and pleasant odour of the compositions. It was found that a conventional (deep) hydrogenation treatment could not provide these beneficial results.

According to a preferred aspect of the invention, the requirements of the Europäisches Arzneibuch 9th Edition, 5th Supplement ("Nachtrag") are fulfilled for the compositions containing renewable isododecane according to the invention described herein.

Yet a further aspect of the present invention is directed to the use of a composition as described herein, obtainable or obtained by the process of the invention as described herein, in cosmetic compositions or applications. The cosmetic compositions or applications in which an isododecane composition may be advantageously used are known in the art and include for example a skin care or hair care product, in particular a make-up product, mascara, lotion, cream, or lip-stick.

### Definitions:

All documents cited herein are incorporated by reference in their entirety for all purposes to the same extent as if each individual document was specifically and individually indicated to be incorporated by reference.

"Renewable" denotes that the carbon content, in particular of the renewable isododecane is from a "new carbon" source. Assessment of the renewably based ("new") carbon content is determined according to ASTM-D6866.

Numerical ranges are provided herein for certain quantities. It is to be understood that these ranges comprise all subranges therein. Thus, the range "from 0.5 to 5" includes all possible subranges therein (e.g., 0.6-5, 0.7-5, 0.5-4.9 etc.) Furthermore, all values within a given range may be an endpoint for the range encompassed thereby.

The use of the word "a" or "an" when used in conjunction with the terms "comprising," "including," "containing," or "having" in the claims and/or the specification may mean "one," but it is also consistent with the meaning of "one or more," "at least one," and "one or more than one."

The terms "about" or "approximately" are defined as being close to as understood by one of ordinary skill in the art. In one non-limiting embodiment, the terms are defined to be within 10%, preferably within 5%, more preferably within 1%, and most preferably within 0.5%.

The phrase "and/or" means and or or. To illustrate, A, B, and/or C includes: A alone, B alone, C alone, a combination of A and B, a combination of A and C, a combination of B and C, or a combination of A, B, and C. In other words, "and/or" operates as an inclusive or.

The terms "wt. %," "vol. %," or "mol. %" refers to a weight, volume, or molar percentage of a component, respectively, based on the total weight, the total volume, or the total moles, respectively, of composition that includes the component. In a non-limiting example, 10 moles of component in 100 moles of the material is 10 mol. % of component. If no other indication is given, "%" or "ppm" refer to "wt. %" or "ppmw", respectively, i.e. the weight of the respective component based on the overall weight or the overall composition that includes the component.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The invention will now be further illustrated, without restriction, by the following Examples.

### Example 1:

A raw isoparaffinic feedstock obtained according to the process described in WO2011/140560A1. It contained:

| | |
|---|---|
| ▪ 99,64 % | isoparaffins |
| ▪ 0,36 % | n-paraffins |
| ▪ 86 % | isododecane (C12), 63% 2,2,4,6,6-Pentamethylheptane |
| ▪ 2 % | C8 isoparaffins |
| ▪ 12 % | C16 isoparaffins |
| ▪ 263 ppmw | aromatics |
| ▪ 0,05% | naphthenes |
| ▪ 0,5 ppm | sulphur |
| ▪ 0,1 ppm | nitrogen |
| ▪ 745g | Br/100 g bromine index (not more than 1000 mg/kg alkenes and alkynes, as defined by bromine index |

Thus, the isoparaffinic feedstock used was in accordance with claims 1, 5 and 6 as attached. It was fractionated under normal pressure to raw isododecane with a boiling range 174°C - 183°C. This raw isododecane fraction (2.5 I) was placed in a round flask of 4 L volume and oleum (2 vol/vol % based on the total weight of the composition; oleum with 20% free mass of SO₃) was added. The flask was placed under a stirrer and stirred at 1500 rpm for 10 minutes. The mixture was transferred to a separation funnel. The spent oleum was separated from the organic phase over a time period of 1 h. Then, the organic phase was transferred back into the round flask, fresh oleum (2 vol/vol % based on the total weight of the composition; oleum with 20% free mass of SO₃) was added and the mixture was stirred for 10 minutes as before. Separation back in the funnel took again about 1 h. The round flask was wetted with 1 vol/vol % water (based on the total volume of the organic phase then added), the organic phase added and stirred for 10 minutes. This small volume of water ensures the separation of sulfonic acids and the paraffin phase. The mixture was transferred into the separation funnel and the aqueous phase was discharged after 2 h (total time from addition of water (step d) to the removal of the aqueous phase (step f) was a bit more than 2 h). Back in the round flask, NaOH solution (0.2 mol) was added (2%), stirred for 10 minutes, then transferred back and separated from the organic phase (1 h). The same procedure was carried out with water. The organic phase was separated, passed over a fluted filter (cellulose), dried and stored in a cool place absent from light.

This isododecane composition ("second composition"), obtained according to the present invention, was highly pure and provided excellent properties as summarized in the Table below. It was in accordance with claims 7 and 8 as attached. It fulfilled the requirements of the Europäisches Arzneibuch 9th Edition, 5th Supplement ("Nachtrag").The properties of the isododecane composition obtained according to the process of present invention were largely superior to those obtained according to the process of WO2011/140560A1 including hydrogenation, even if the hydrogenation step was repeated.

### Example 2

This Example differs from Example 1 in that the time period between the addition of water according to step d) and the removal of the aqueous phase was about 20 hours. The raw isoparaffinic feedstock was fractionated under normal pressure to raw isododecane with a boiling range 174°C - 183°C. The resulting (fractionated) composition ("raw isododecane fraction") (same as in Example 1 above; 2.5 I) was placed in a round flask of 4 L volume and oleum (2 vol/vol % based on the total weight of the composition; oleum with 20% free mass of SO₃) was added. The flask was placed under a stirrer and stirred at 1500 rpm for 10 minutes. The mixture was transferred to a separation funnel. The spent oleum was separated from the organic phase over a time period of 1 h. Then, the organic phase was transferred back into the round flask, fresh oleum (2 vol/vol % based on the total weight of the composition; oleum with 20% free mass of SO₃) was added and the mixture was stirred for 10 minutes as before. Separation back in the funnel took again about 1 h. The round flask was wetted with 1 vol./vol. % water (based on the total volume of the organic phase then added), the organic phase added and stirred for 10 minutes. The mixture was transferred into the separation funnel and the aqueous phase was discharged after 20 to 24 h (total time from addition of water (step d) to the removal of the aqueous phase (step f) was at least a bit more than 20 h). Back in the round flask, NaOH solution (0.2 mol) was added (2%), stirred for 10 minutes, then transferred back and separated from the organic phase (1 h). The same procedure was carried out with water.

The organic phase was separated, passed over a fluted filter (cellulose), dried and stored in a cool place absent from light.

This isododecane composition ("second composition"), obtained according to the present invention, was highly pure and provided excellent properties as summarized in the Table below. It was in accordance with claims 7 and 8 as attached. It fulfilled the requirements of the Europäisches Arzneibuch 9th Edition, 5th Supplement ("Nachtrag").

The properties of the isododecane composition obtained according to the process of present invention were largely superior to those obtained according to the process of WO2011/140560A1 including hydrogenation, even if the hydrogenation step was repeated.

It was surprisingly even superior to the results obtained in Example 1, due to the longer time period between the addition of water according to step d) and the removal of the aqueous phase according to step f) of more than 20 hours.

The results of the analysis of the products according to Examples 1 and 2, respectively, are summarized in the following Table.

| Parameter | Method | Raw isoparafinic feedstock | Product of Example 1 | Product of Example 2 (overnight treatment)) |
|---|---|---|---|---|
| Colour (Saybolt) | ASTM D156 | 23 | 30 | 30 |
| Color (Hazen) | ASTM D1209 | 38 | 4 | 3 |
| Odour | Internal odor panel (*) | paraffinic | lightly paraffinic | Odorless, light, even more pleasant than product of Example 1 |
| Bromine index | DIN EN 51774 | 745 mg Br/100 g | 33 mg Br/ 100 g | 33 mg Br/100 g |
| Aromatics (UV) | UOP 495 | 263 ppm | 19,7 ppm | < 5 ppm |
| Sulfur** | ASTM D5453 | 5 ppm | < 5 ppm | < 5 ppm |
| Nitrogen** | DINEN 51451 | 0,1 ppm | <0,1 ppm | <0,1 ppm |

| | | | | |
|---|---|---|---|---|
| (*) A panel of 5 persons, trained in the blind testing and categorized description of the odour of paraffinic compositions, performed a blind test of the respective samples (2 ml in a glass beaker, one drop each spread on filter paper). The testing was performed in triplicates for each sample. (**) The raw isoparafinic feedstock used in the Examples had already very low sulfur and nitrogen content - higher sulfur and nitrogen contents are often present in raw isoparaffinic feedstock, and the reduction thereof by the procedd according to the invention is even more pronounced. The data for Examples 1 and 2 also show that no sulfur contamination was added despite the Oleum treatment. As evident from the above Table, while the product of Example 1 provides much superior analysis results as compared to the product of the prior art, the product of Example 2 has unexpectedly further improved properties. | | | | |

## Claims

1. A process for treating a composition containing renewable isododecane, the process comprising the following steps:
a) Selecting a first composition containing at least 20% renewable isododecane;
b) Optionally increasing the content of renewable isododecane in the first composition to at least 80%, in particular at least 85%;
c) Treating the first composition with Oleum;
d) Adding water to the treated first composition of step c) and agitating the mixture;
e) Allowing the aqueous phase containing oxidized and/or sulfonized compounds and the non-aqueous phase containing renewable isododecane to separate;
f) Removing the aqueous phase containing oxidized and/or sulfonated compounds in order to obtain the non-aqueous phase containing the renewable isododecane;
g) Adding an alkaline aqueous washing solution to the non-aqueous phase containing the renewable isododecane;
h) Removing the aqueous washing solution of step g), and optionally drying the non-aqueous phase containing the renewable isododecane in order to obtain a second composition containing renewable isododecane;
i) Optionally repeating steps c) to h) using the non-aqueous phase containing the renewable isododecane obtained in step h).

2. The process of any of the preceding claims, wherein the time period between the addition of water according to step d) and the removal of the aqueous phase according to step f) is at least 5 hours, preferably at least 10 hours, in particular at least 15 hours, more particular at least 20 hours.

3. The process of any of the preceding claims, wherein 0.1 to 5 %, in particular 0.5 to 5 % of Oleum are used in step c), based on the total mass of the first composition.

4. The process of claim 1 wherein the Oleum has at least 5%, preferably at least 10%, in particular about 20% free mass of SO₃.

5. The process of any of the preceding claims, wherein the first composition comprises:
at least 95% isoparaffins,
not more than 5% n-paraffins,
at least 40%, preferably at least 50% isododecane,
not more than 10% C8 isoparaffins,
not more than 40% C16 isoparaffins,
not more than 5%, preferably not more than 3%, in particular not more than 1% aromatics,
not more than 1% naphthenes,
no more than 5%, preferably not more than 3%, in particular not more than 1% hydrocarbons comprising heteroatoms, in particular comprising oxygen, nitrogen or sulphur,
not more than 2 wt.-%, preferably less than 1 wt.-%, in particular less than 2000 mg/kg alkenes and alkynes, as defined by bromine index.

6. The process according to claim 5, wherein the isododecane present in the first composition comprises at least 60%, preferably at least 70%, in particular at least 75% 2,2,4,6,6-Pentamethylheptane.

7. The process of any of the preceding claims, wherein the second composition comprises:
less than 200 ppm, in particular less than 50 ppm aromatics
less than 200ppm hydrocarbons comprising heteroatoms, in particular comprising oxygen, nitrogen or sulfur;
less than 1 ppm sulfur
no more than 1% hydrocarbons comprising heteroatoms, in particular comprising oxygen, nitrogen or sulfur;
less than 600 mg/kg alkenes and alyknes, as defined by bromine index.

8. The process according to any of the preceding steps, further comprising one or more distillation step, wherein the second composition comprises
at least 98% isoparaffins,
not more than 2% n-paraffins,
less than 1% C8 isoparaffins,
at least 80% isododecane, preferably at least 98% thereof 2,2,4,6,6-Pentamethylheptane,
not more than 2% C16 isoparaffins,
not more than 2% paraffins C11 or smaller,
not more than 1% naphthenes, in particular less than 50 ppm.

9. The process according to any of the preceding claims, wherein the first composition is a renewable jet fuel blendstock, in particular having the composition according to claim 5.

10. The process according to any of the preceding claims, wherein the amount of water added in step d) is less than 5 vol./vol. %, preferably less than 3 vol./vol. %, in particular within 0.01 to 5 vol./vol. % of the treated first composition of step c).

11. A composition containing renewable isododecane, obtainable by the process according to any of claims 1 to 10.

12. Use of a composition according to claim 11 or prepared according to the process of any of claims 1 to 10 in cosmetic or pharmaceutical compositions or applications.

13. The use according to claim 12 wherein the cosmetic product or application is a skin care or hair care product, in particular a make-up product, mascara, lotion, cream, or lip-stick.
